Europäisches Patentamt

European Patent Office

Office européen des brevets

(19)

(11) Publication number : **0 397 595 B1**

# EUROPEAN PATENT SPECIFICATION

(12)

(45) Date of publication of patent specification :
**14.12.94 Bulletin 94/50**

(51) Int. Cl.⁵ : **G01N 33/50, // G01N33/52**

(21) Application number : **90810282.5**

(22) Date of filing : **09.04.90**

(54) **A method for the evaluation of antiallergic substances.**

(30) Priority : **10.04.89 JP 91578/89**

(43) Date of publication of application :
**14.11.90 Bulletin 90/46**

(45) Publication of the grant of the patent :
**14.12.94 Bulletin 94/50**

(84) Designated Contracting States :
**AT BE CH DE DK ES FR GB GR IT LI LU NL SE**

(56) References cited :
**EMBASE, 1988; R. NISHIOKA et al., no. 88251997
CHEMICAL ABSTRACTS, vol. 109, no. 7, 15 August 1988, Columbus, OH (US); H. KURIKI et al., p. 35, no. 48024g
EMBASE, 1985; T. HATA et al., no. 85088184
EMBASE, 1984; H. KUMAZAWA et al., no. 84241642
EMBASE, 1987; N. YAGI et al., no. 87105429
ACTA OTOLARYNGOL., vol. 103, 1987; N. YAGI et al., pp. 312-317**

(73) Proprietor : **NIPPON ZOKI PHARMACEUTICAL CO. LTD.
10, 2-chome Hiranomachi
Higashi-ku
Osaka (JP)**

(72) Inventor : **Namimatsu, Akio, c/o Instit. of Bio-Active Science
Nippon Zoki Pharmaceutical Co., Ltd.
442-1,Kinashi
Yashiro-cho, Katoh-gun, Hyogo (JP)**
Inventor : **Go, Kouichiro, c/o Instit. of Bio-Active Science
Nippon Zoki Pharmaceutical Co., Ltd.
442-1,Kinashi
Yashiro-cho, Katoh-gun, Hyogo (JP)**

(74) Representative : **Kerr, Andrew
A.KERR AG,
Postfach,
Finkelerweg 44
CH-4144 Arlesheim (CH)**

EP 0 397 595 B1

## Description

The present invention relates to a method for the evaluation of antiallergic substances, in which nasal secretion is quantitatively measured using experimental (laboratory) animal models having nasal mucosal hypersensitivity.

The method can be used to conduct accurate quantitative analysis of drug efficacy in the pharmacological studies of various substances, in particular screening tests of substances for the purpose of pharmaceutical research and development.

Methods for assessing nasal mucosal sensitivity for the evaluation of antiallergic substances are already known, one of these methods being based on measuring nasal secretion. However, when employing small test animals such as rats or guinea pigs, it is difficult to quantitatively measure the amount of nasal secretion. Quantitative subjective evaluation has indeed frequently been employed, but it has been found hard to give great weight to the results.

It is an object of the present invention to provide a method of evaluating antiallergic substances, which is characterized by measuring the amount of nasal secretion quantitatively using experimental (laboratory) animal models possessing nasal mucosal hypersensitivity.

Chemical Abstracts, 1988, 109, Abstract no. 48024g describes the effect of amlexanox on experimental allergic rhinitis in "actively sensitized" rats and guinea pigs. $I_gE$-mediated increase in nasal capillary permeability was measured in rats, and $I_gE$-mediated increase in nasal resistance in guinea pigs.

EMBASE (1988) Abstract no. 88251997 [Nishioka et al. Pract. Otol.,1988, 81/10, pp 1437-1441], and EMBASE (1987) Abstract no. 87105429 [Yagi et al. Acta Otolaryngol., 1987, 103, pp 312-317] describe the use of nasal threads to measure nasal secretion in normal and allergic human patients.

EMBASE (1985) Abstract no. 85088184 [Hata et al. Jpn. J. Psychosom. Med., 1984, 24/4, pp 301-308] describes the influence of various drugs on the immune response of SART stressed animals.

According to the invention there is provided a method of evaluating an antiallergic substance by measurement of nasal secretion in laboratory animals, characterised in that there are employed one or more laboratory animals with nasal mucosal hypersensitivity, the said animals having been subjected to nasal provocation with an allergen to induce nasal secretion and wherein measurement of the relative degree of nasal secretion of the animals is conducted utilising one, or a plurality of nasal threads used together.

The evaluation according to this invention includes both quantitative and qualitative testing.

The efficacy of antiallergic substances can be more precisely evaluated using experimental (laboratory) animal models having nasal mucosal hypersensitivity than by using normal animals. It is preferred to use stress-loaded animals which have been stressed by changes in environmental rhythm, or animals having enhanced nasal mucosal sensitivity prepared by repeated nasal provocation with an allergen in actively sensitized animals.

Stress as a result of change of rhythm in environmental temperature (SART stress: Specific Alternation of Rhythm in environmental Temperature - stress) is loaded by maintaining experimental animals in a specific stressed condition at an environmental temperature which varies between low temperature (from -3°C to 8°C) and ambient temperature (from 20°C to 25°C) mutually. Stress of differing intensity may be loaded by varying the environmental conditions, e.g. the low temperature and the ambient temperature or the loading period. SART-stressed animals can in general be prepared according to the method of Kita et al [Folia pharmacol. japon., 71, 195-210 (1975)].

In SART-stressed animals, abnormalities of vital functions such as gradual decline of body weight gain, a lowering of sensitivity to acetylcholine of the isolated duodenum, an increase of respiration and heart rate, a prolongation of the QRS time and the like have been reported. Pathological studies of SART-stressed animals have now shown that SART-stressed animals show a significant increase in nasal secretion and nasal mucosal hypersensitivity by nasal provocation with an allergen after passive sensitization.

Animals showing nasal mucosal hypersensitivity resulting from repeated nasal provocation with an allergen in actively sensitized animals can be employed as experimental (laboratory) animal models with nasal mucosal hypersensitivity in the present invention.

Active sensitization with an allergen can be carried out by conventional methods commonly employed in this field, for example, by administering an allergen such as ovalbumin, DNP-ascaris or DNP-bovine serum albumin to experimental laboratory animals at least three times at suitable intervals from one week to one month, if necessary, together with an adjuvant.

Experimental (laboratory) animal models possessing nasal mucosal hypersensitivity can be prepared by repeated nasal provocation with an allergen in actively sensitized animals. Conventional methods for inducing nasal secretion, for example, administering an allergen or a chemical mediator such as histamine, methacholine or acetylcholine intranasally can be employed for inducing nasal secretion in these experimental (laboratory)

2

animal models with nasal mucosal hypersensitivity.

The above mentioned method for preparing experimental (laboratory) animal models having nasal mucosal hypersensitivity by repeated nasal provocation with an allergen in actively sensitized animals requires a long period of sensitization. But the procedures such as a sensitization method and the like are very simple, and the obtained animal models with nasal mucosal hypersensitivity have the advantage of being used repeatedly in the examination with intervals of no drug.

A method has now been found which can provide clear evaluation concerning the inhibitory action of drugs for nasal secretion by using the said experimental (laboratory) animal models with nasal mucosal hypersensitivity, though the inhibitory action could not be measured clearly by using normal animals.

In this specification the animals which can be used are variously referred to as experimental animal models and laboratory animals. Examples of such common experimental animals are, for example, rats, mice, guinea pigs, hamsters, rabbits, monkeys such as common tupia and the like, which can be used as the animal employed in the method of the present invention for evaluating antiallergic substances. It is preferred to use guinea pigs or hamsters since these are readily available and are also particularly suitable from the standpoint of several features such as the size, shape and the like, of the nose.

The data which do not vary so widely can be obtained by using animals with substantially equal nasal mucosal sensitivity, so it is preferable to give attention to the previous measurement of the threshold of sensitivity to histamine, methacholine or acetylcholine, i.e. selecting animals having substantially equal threshold of sensitivity to the chemical mediator, and classifying each given group with substantially equal average threshold.

In SART-stressed animals, the method of administering histamine, methacholine and the like intranasally and the method of nasal provocation with an allergen in actively or passively sensitized animals is employed as the method for inducing nasal secretion. Actually, there are a great many patients suffering from nasal allergy induced by house dust, Japanese cedar pollen and the like. Since the method using allergen induction is similar to the total biological response system, this is a practical and reasonable one as the method for evaluating antiallergic substances. In particular, the passive sensitization method is simple, can be carried out for a short term, and is thus preferable because the data do not vary so widely, and the process has good reproducibility.

In order to induce nasal secretion in passively sensitized animals with an allergen, firstly, antiserum to the allergen is prepared, and secondly, the nasal secretion is induced by administering an allergen intranasally after sensitization by injecting the antiserum to test animals. The method for the preparation of antiserum and the passive sensitization method with antiserum can be similarly carried out according to the general manner employed commonly. Allergens such as ovalbumin, DNP-ascaris and DNP-bovine serum albumin commonly used in this field can be employed.

By using animal models with nasal mucosal hypersensitivity, the efficacy of antiallergic substances can be evaluated by the passive sensitization method with diluted antiserum, and the evaluation test can be carried out with only a small amount of antiserum. This method thus has economic advantages.

As for the method for measuring nasal secretion, there are several methods, for example, making a rhinorrhea symptom score by a tester's observation, collecting nasal secretion by inserting an aspiration tube into the nostril, absorbing nasal secretion with utilizing a filtration paper and defatted cotton thread to measure the amount of it by weight, measuring the stretch of color due to nasal secretion utilizing a thread dyed with fluorescein and the like.

In case of application of the said methods to small experimental animals, the method of collecting nasal secretion by means of an aspiration tube and that of utilizing a filtration paper are difficult to put into practice because of very small nostrils and very little nasal secretion in these animals. Because the method of making a rhinorrhea symptom score by a tester's observation is not objective, it is thus hard to say that this method is a quantitative one. Thus, one essential aspect of the invention is the method of utilizing a nasal thread, as the method for evaluating antiallergic substances using small experimental animals, because it is adaptable even to a slight amount of nasal secretion, can be used with little irritation to nasal mucosa, and can objectively express the amount of nasal secretion numerically. The thread for the measurement of the amount of nasal secretion can be prepared simply, for example, by suitable treatment such as defatting commercial cotton thread, if necessary, and then dyeing the defatted thread with a fluorescein solution. The procedure for measuring the amount of nasal secretion is very easy to perform by measuring the stretch of fluorescein color due to nasal secretion, or measuring the weight of defatted thread absorbing nasal secretion.

EXAMPLE

(1) Method for loading SART stress.

Male Hartley strain guinea pigs weighing 300 to 500 g were used. The threshold of nasal mucosal sensitivity to methacholine of these animals was previously measured using nasal thread dyed with fluorescein, and these guinea pigs, having normal sensitivity, were divided into groups of 7 or 8 animals to make the average of the threshold of sensitivity substantially equal in each group. To load SART stress, guinea pigs were kept in a thermostatic box at the environmental temperature of 0°C and 24°C alternately for one hour periods from 10:00 a.m. to 5:00 p.m., and at 0°C from 5:00 p.m. to 10:00 a.m. the next day. This stress loading was continued for 5 days prior to the following sensitization.

(2) Preparation of anti-ovalbumin serum.

Guinea pigs were sensitized intraperitoneally using 20 g of ovalbumin and 10 mg of aluminium hydroxide (adjuvant) in 1 ml of saline 7 times every 2 weeks. After 2 weeks of the sensitization, airway sensitization was performed by ultranebulization of 3 ml of 0.01 % ovalbumin in saline for 5 consecutive days. One week later, nasal provocation was performed by application of 50 1 of 1 % ovalbumin in saline on both anterior nares, and antiserum was collected from guinea pigs showing typical nasal symptoms of nasal allergy such as sneeze, nasal secretion and asthma. This anti-ovalbumin serum can be preserved at -80°C.

(3) Nasal provocation in passively sensitized guinea pigs.

Provocation was made by application of 50 l of 1 % ovalbumin on the left anterior naris 2 days after passive sensitization by injecting 2 ml of diluted anti-ovalbumin serum (diluted ratio; x 1/8) intraperitoneally, and nasal secretion was measured. Passive sensitization in SART-stressed guinea pigs was made 3 days after the loading SART stress.

(4) Active sensitization.

Active sensitization can be carried out in the similar manner of the preparation of anti-ovalbumin serum mentioned above. Namely, 1 g ovalbumin and 5 mg of aluminium hydroxide (adjuvant) in 1 ml of saline were injected into guinea pigs intraperitoneally 6 times every 2 weeks.

(5) Repeated nasal provocation with an allergen to enhance nasal mucosal sensitivity.

Two weeks after active sensitization with ovalbumin, 50 l of 1 % ovalbumin in saline were administered into both anterior nares to initiate nasal provocation with an allergen. Then nasal provocation with ovalbumin was repeated 1, 4, 7, 10 and 13 days after the first provocation.

In the above mentioned passive or active sensitization and nasal provocation with an allergen, the amount and species of allergens may be suitably chosen.

(6) Measurement of nasal secretion

Defatted cotton threads of No. 40/2 were dyed with 10% fluorescein-Na in physiological saline at one end, and were cut to leave a total length of 100 mm and a colored length of 10 mm. About 15 mm of the colored end of the thread were inserted into the left anterior naris for 60 sec. The length of thread dyed with fluorescein due to nasal secretion was regarded as the amount of nasal secretion. A few threads can be used together according to the amount of nasal secretion.

The relationship between the stretch of color of a thread dyed with fluorescein and the amount of nasal secretion was studied. As a result of the study, it was clearly shown that the stretch of color was proportional to the increased weight of the thread.

(l) The activity of the antiallergic substance was evaluated by using passively sensitized SART-stressed guinea pigs according to the said methods (1) to (3) and (6) in the Example. 0.3 to 3.0 mg/kg/day of Ketotifen fumarate as a test drug were orally administered.

An example of the results is shown in Table 1. The amount of nasal secretion was represented by the stretch of color of a thread dyed with fluorescein.

Table 1

| | Nasal secretion (mm) | Inhibition (%) |
|---|---|---|
| Normal guinea pig | 23.3 | − |
| Control | 45.0 | 0 |
| Ketotifen (0.3 mg) | 33.5 | 25.5 |
| (1.0 mg) | 23.9 | 46.9 |
| (3.0 mg) | 16.1 | 64.2 |

(II) Enhanced nasal mucosal sensitivity was measured using animals prepared by repeated nasal provocation with ovalbumin in actively sensitized guinea pigs with an allergen according to methods (4) to (6) in the Example. Inhibitory action for nasal secretion of Ketotifen fumarate (1.0 mg/kg/day, p.o.) was also measured in this assay system.

An example of the results is shown in Table 2.

Table 2

| Provocation with allergen | Nasal secretion (mm) | |
| --- | --- | --- |
| | No test drug | Ketotifen |
| 1st | 68.8 | 68.9 |
| 2nd (1 day) | 123.3 | 54.1 |
| 3rd (4 days) | 146.6 | 73.0 |
| 4th (7 days) | 152.9 | 68.5 |
| 5th (10 days) | 187.3 | 77.3 |
| 6th (13 days) | 168.1 | 96.9 |

As shown by the results set out in Table 1, inhibitory action on nasal secretion of the antiallergic substance can be measured quantitatively by measuring the amount of nasal secretion by the use of a nasal thread dyed with fluorescein in experimental (laboratory) animal models with nasal mucosal hypersensitivity, which are prepared by loading with SART stress. When using Ketoprofen fumarate as the test drug, dose-dependent inhibitory action on nasal secretion was observed at doses of 0.3 to 3.0 mg/kg/day (p.o.).

Also as shown by the result in Table 2, experimental (laboratory) animal models possessing nasal mucosal hypersensitivity can be prepared by repeated nasal provocation in actively sensitized animals. In addition, nasal sensitivity to methacholine was investigated based on the amount of nasal secretion in this assay system by giving methacholine (10 mol) intranasally one day after each provocation with an allergen. This investigation showed that nasal sensitivity to methacholine was enhanced by repeated nasal provocation with an allergen. Thus the obtained animals can be employed as an environmental animal model with nasal mucosal hypersensitivity as well as SART-stressed animals.

The efficacy of antiallergic substances can thus be measured dose-dependently, so that the effective amount of the drug can be expressed numerically, such as $ED_{50}$ etc.

On the contrary, when using normal animals, the inhibitory action on nasal secretion was not clearly measured, and so the activity of antiallergic substances could not be evaluated quantitatively.

Nasal mucosal sensitivity is often enhanced in patients suffering from nasal allergy. The method of the present invention, which is characterized by utilizing experimental (laboratory) animal models possessing nasal mucosal hypersensitivity, is preferably practical and reasonable as it is carried out under the experimental condition close to the diseased condition of actual human patients and clinical feature.

The reproducibility of the method of the present invention was studied. Thus, both in the cases of loading SART stress and repeating nasal provocation using an allergen in actively sensitized animals, the experimental (laboratory) animal models with nasal mucosal hypersensitivity can be prepared reproducibly.

The experimental (laboratory) animal models with nasal mucosal hypersensitivity by repeated nasal provocation with an allergen in actively sensitized animals have the advantage that these animals can be used

EP 0 397 595 B1

repeatedly in the examinations with intervals of no drug.

And in case of SART-stressed animals with nasal mucosal hypersensitivity, it is possible to evaluate antiallergic substances in a simple operation, in a short time and without the data varying so widely.

The method of utilizing nasal threads is the most easy, simple and preferable one providing the quantitative measurement of the amount of nasal secretion of small experimental (laboratory) animals.

As mentioned above, the method of the present invention utilizing experimental (laboratory) animal models with nasal mucosal hypersensitivity is practical, can make objective and clear evaluations and express the effective amount of drugs numerically, such as $ED_{50}$, has reproducibility, gives the data without varying so widely, can be carried out in a simple procedure and in a short time, and has economic advantages. Therefore, in particular, this method is very useful as the method for measuring the activity of antiallergic substances in the field of nasal allergy treatment, for example, screening for the purpose of research and development into new antiallergic drugs, or the standardization method for such drugs.

## Claims

1. A method of evaluating an antiallergic substance by measurement of nasal secretion in laboratory animals, characterised in that there are employed one or more laboratory animals with nasal mucosal hypersensitivity, the said animals having been subjected to nasal provocation with an allergen to induce nasal secretion and wherein measurement of the relative degree of nasal secretion of the animals is conducted utilising one, or a plurality of nasal threads used together.

2. A method according to Claim 1 wherein said animals have been stress-loaded by changing their environmental rhythm.

3. A method according to Claim 2 wherein said animals have been stress-loaded by alternation of environmental temperature.

4. A method according to any preceding claim wherein said animals have been subjected to passive sensitization.

5. A method according to any preceding claim wherein said animals have been subjected successively to SART and passive sensitization.

6. A method according to Claim 1 wherein said animals have been subjected to active sensitization followed by repeated nasal provocation to enhance nasal mucosal sensitivity.

7. A method according to any preceding claim wherein a measurement is made of the length of elution of a marker substance in the thread portion(s) inserted into the nostril of the animal.

8. A method according to Claim 7 wherein the marker is a fluorescent dye.

9. A method according to any preceding claim wherein said animals are guinea pigs.

10. A method according to any preceding claim for qualitatively comparing the performance of a plurality of antiallergic substances.

11. A method according to any preceding claim of quantitatively determining the performance of a said antiallergic substance.

## Patentansprüche

1. Verfahren zur Evaluierung von antiallergischen Substanzen durch Messen der Nasensekretion bei Labortieren, dadurch gekennzeichnet, daß ein oder mehrere Labortiere mit einer Hypersensibilität der Nasenschleimhaut verwendet werden, wobei die Tiere zur Induzierung einer Nasensekretion einer Reizung der Nasenschleimhäute mit einem Allergen unterzogen wurden und wobei das relative Ausmaß der Nasensekretion der Tiere mit Hilfe eines oder mehrer Fäden gemessen wird.

**2.** Verfahren nach Anspruch 1, wonach bei den Tieren durch Verändern ihres Umweltrhythmus eine Streßsituation hergestellt wurde.

**3.** Verfahren nach Anspruch 2, wonach bei den Tieren durch Verändern der Umgebungstemperatur eine Streßsituation hergestellt wurde.

**4.** Verfahren nach einem der vorstehenden Ansprüche, wonach die Tiere einer passiven Sensibilisierung unterzogen wurden.

**5.** Verfahren nach einem der vorstehenden Ansprüche, wonach die Tiere erst einer SART-Streßsituation und dann einer passiven Sensibiliserung unterzogen wurden.

**6.** Verfahren nach Anspruch 1, wonach die Tiere einer aktiven Sensibilisierung unterzogen wurden und danach einer Mehrfachreizung der Nasenschleimhäute, um deren Sensibilität zu erhöhen.

**7.** Verfahren nach einem der vorstehenden Ansprüche, wonach die Länge der Eluierung einer Markierungssubstanz auf dem in die Nasenöffnung des Tieres eingeführten Fadens gemessen wird.

**8.** Verfahren nach Anspruch 7, wonach der Marker ein fluoreszierender Farbstoff ist.

**9.** Verfahren nach einem der vorstehenden Ansprüche, wonach es sich bei den Tieren um Meerschweinchen handelt.

**10.** Verfahren nach einem der vorstehenden Ansprüche, wonach die Leistung einer Vielzahl von antiallergischen Substanzen qualitativ verglichen wird.

**11.** Verfahren nach einem der vorstehenden Ansprüche, wonach die Leistung einer Vielzahl von antiallergischen Substanzen quantitativ verglichen wird.


**Revendications**

**1.** Une méthode d'évaluation d'une substance anti-allergique par la mesure de la sécrétion nasale chez des animaux de laboratoire, caractérisée en ce qu'on utilise un ou plusieurs animaux de laboratoire présentant une hypersensibilité de la muqueuse nasale, lesdits animaux ayant été soumis à un test de provocation nasale par un allergène dans le but de déclencher une sécrétion nasale, et dans laquelle on mesure le degré relatif de sécrétion nasale des animaux au moyen d'un ou de plusieurs fils insérés dans les narines utilisés conjointement.

**2.** Une méthode selon la Revendication 1 dans laquelle lesdits animaux ont été soumis à des contraintes de stress par modification du rythme de leur environnement.

**3.** Une méthode selon la Revendication 2 dans laquelle lesdits animaux ont été soumis à des contraintes de stress par variation de la température ambiante.

**4.** Une méthode selon l'une quelconque des Revendications précédentes dans laquelle lesdits animaux ont été soumis à une sensibilisation passive.

**5.** Une méthode selon l'une quelconque des Revendications précédentes dans laquelle lesdits animaux ont été soumis successivement à une sensibilisation par variation spécifique du rythme de la température ambiante (SART) et à une sensibilisation passive.

**6.** Une méthode selon la Revendication 1 dans laquelle lesdits animaux ont été soumis à une sensibilisation active suivie d'un test de provocation nasale répété dans le but d'accroître la sensibilité de la muqueuse nasale.

**7.** Une méthode selon l'une quelconque des Revendications précédentes dans laquelle on mesure la longueur d'élution d'une substance de marqueur dans la ou les portion(s) de fils insérée(s) dans la narine de l'animal.

8. Une méthode selon la Revendication 7 dans laquelle le marqueur est un colorant fluorescent.

9. Une méthode selon l'une quelconque des Revendications précédentes dans laquelle lesdits animaux sont des cochons d'Inde.

10. Une méthode selon l'une quelconque des Revendications précédentes permettant de comparer qualitativement les performances d'une pluralité de substances anti-allergiques.

11. Une méthode selon l'une quelconque des Revendications précédentes permettant de déterminer quantitativement les performances d'une dite substance anti-allergique.